# FASCICULE DE BREVET EUROPEEN

(11) **EP 1 453 443 B1**
(45) Date de publication et mention de la délivrance du brevet: **13.01.2010**
(21) Numéro de dépôt: 02788299.2
(22) Date de dépôt: 09.12.2002
(51) Int. Cl.: A61F 2/42

(54) **IMPLANT TRAPEZIEN OU TRAPEZO-METACARPIEN**
TRAPEZIUM ODER TRAPEZIO-METAKARPALES IMPLANTAT
TRAPEZIUM OR TRAPEZOMETACARPAL IMPLANT

(30) Priorité: 12.12.2001 FR 0116091
(43) Date de publication de la demande: 08.09.2004
(73) Titulaire: BIOPROFILE, 38024 Grenoble Cedex 1 (FR)
(72) Inventeur: HASSLER, Michel, F-38330 Saint-Ismier (FR); REAL, Cécile, F-38000 Grenoble (FR); PEQUIGNOT, Jean-Pierre, F-06000 Nice (FR); ALLIEU, Yves, F-34070 Montpellier (FR)
(74) Mandataire: Micheli & Cie SA
(86) Numéro de dépôt international: PCT/IB2002/005189
(87) Numéro de publication internationale: WO 2003/049651

(56) Documents cités:
- DE-A- 19 925 529
- DE-U- 29 721 522
- FR-A- 2 680 967
- US-A- 3 745 590
- US-A- 3 924 276

## Description

### DOMAINE DE L'INVENTION

La présente invention concerne un implant trapézien ou trapézo-métacarpien, comprenant une tige terminée par une tête. La tige est destinée à être insérée dans le métacarpe du pouce, et la tête à se loger dans un espace obtenu notamment en retirant la totalité du trapèze, dans le cas d'un implant trapézien, ou une partie du trapèze, dans le cas d'un implant trapézo-métacarpien.

L'implant selon l'invention peut être utilisé pour traiter la rhizarthrose, affection très fréquente consistant dans une arthrose de la base du pouce, essentiellement trapézo métacarpienne.

### ETAT DE LA TECHNIQUE

Il existe plusieurs types d'implants trapéziens et trapézo-métacarpiens.

Parmi les plus connus figure l'implant trapézien dit de Swanson, constitué d'une tige terminée par une tête, le tout réalisé en silicone. Dans cet implant, la tête a une taille et une forme adaptées pour remplacer la totalité du trapèze.

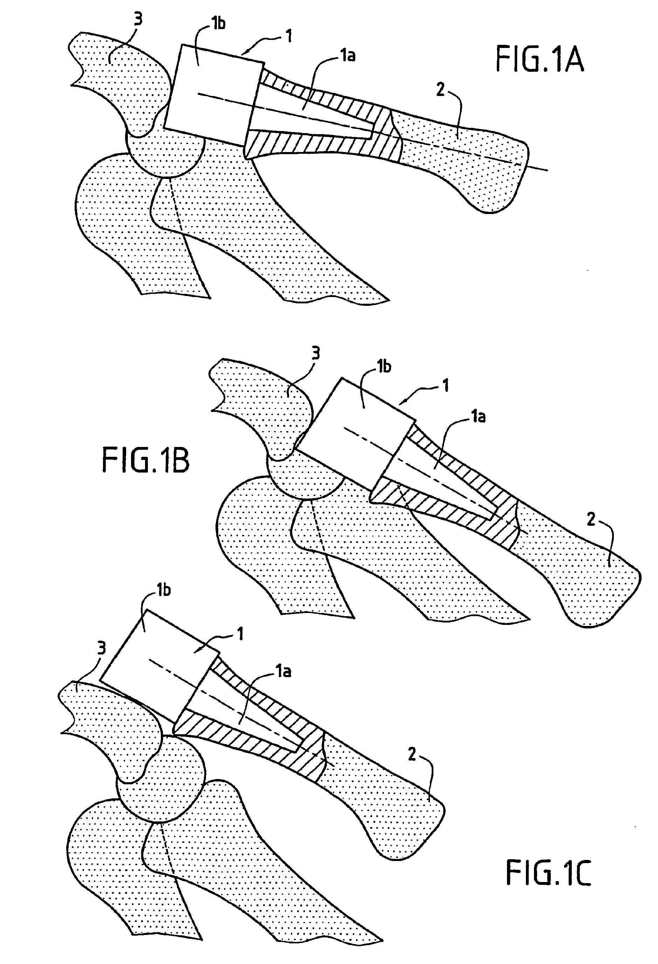

Cet implant présente deux inconvénients principaux :
- la matière dans laquelle il est fabriqué est inadaptée : le silicone provoque chez certains patients une allergie connue sous le nom de siliconite ; en outre, du fait de sa trop faible dureté, le silicone s'use au contact des os avoisinants, entraînant une détérioration de l'implant;
- il n'est pas stable et, en particulier, a une tendance à se luxer ou se subluxer (c'est-à-dire se luxer partiellement) ; les figures 1A, 1B et 1C illustrent un implant trapézien 1, dont la tige 1a est montée dans le métacarpe 2 et la tête 1b remplace complètement le trapèze ; à la figure 1A, l'implant est normalement positionné par rapport aux os avoisinants et notamment par rapport au scaphoïde 3 avec lequel il est en contact ; la figure 1B montre une configuration typique de subluxation de l'implant lors d'un effort de pince impliquant le pouce et au moins un autre doigt de la main ; la figure 1C montre une configuration typique de luxation de l'implant après un tel effort de pince.

Afin d'accroître la stabilité de cet implant et éviter qu'il ne se subluxe comme montré à la figure 1B, ou qu'il ne se luxe comme montré à la figure 1C, il a été proposé de perforer sa tête de part en part pour y faire passer une languette tendineuse apte à limiter ses mouvements, ou de le ligaturer, comme décrit dans les brevets américains US 3 745 590 et US 3 924 276. Ces techniques compliquent la tâche du chirurgien et brident la mobilité de l'implant, causant une gène pour le patient.

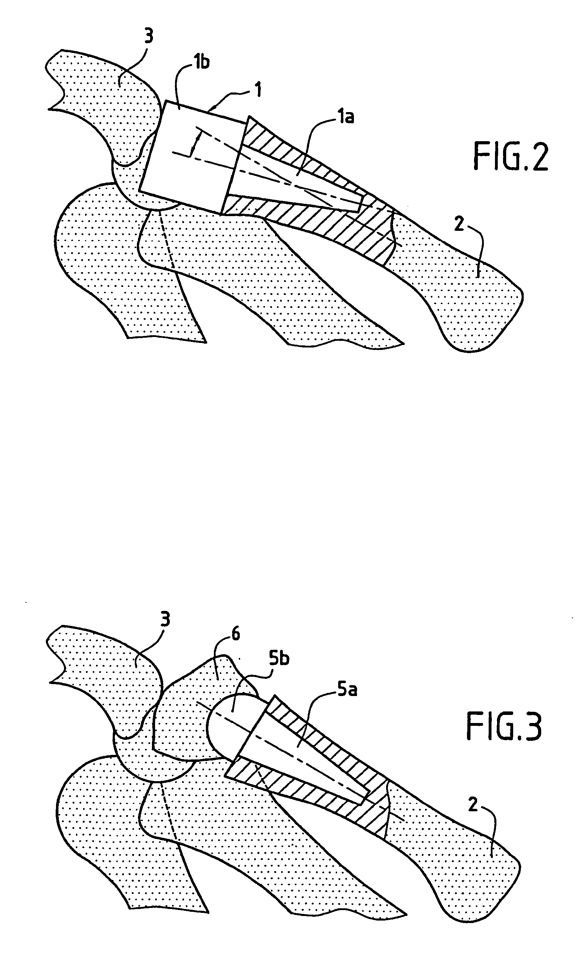

Une autre solution est décrite dans l'article intitulé "L'implant trapézien de Swanson dans le traitement de l'arthrose péri-trapézienne", de Y. Allieu et al., paru dans la revue Annales de Chirurgie de la Main, Volume 3, No 2, 1984. Elle consiste à effectuer préalablement une coupe oblique dans l'extrémité proximale du métacarpe, et à fixer l'implant en position de varus, c'est-à-dire à le fixer de façon inclinée par rapport à l'axe du métacarpe en l'orientant vers l'intérieur de la main, comme illustré à la figure 2. Ainsi incliné, l'implant est maintenu en contact avec le scaphoïde, et a moins tendance à se luxer ou se subluxer. Cette solution améliore donc significativement la stabilité de l'implant. Néanmoins, fixer l'implant de façon inclinée par rapport à l'axe du métacarpe oblige à pratiquer un trou oblique dans la fût du métacarpe, geste chirurgical délicat à réaliser qui nécessite de creuser dans la partie périphérique dure de l'os et qui en outre fragilise ce dernier.

On connaît également des implants trapézo-métacarpiens, tels que l'implant condylien de Swanson, dont la tête vient se loger dans un espace obtenu d'une part en pratiquant une coupe sur l'extrémité proximale du métacarpe et d'autre part en retirant une partie du trapèze opposée à cette extrémité proximale. Ces implants sont aussi réalisés en silicone. Une version en titane de l'implant condylien de Swanson a toutefois été proposée.

De même que pour l'implant trapézien de Swanson, la matière dans laquelle sont fabriqués ces implants trapézo-métacarpiens est inadaptée. Comme expliqué précédemment, le silicone est trop mou. Le titane, lui, est trop dur et use les os avec lesquels il est en contact. Il rend en outre l'implant douloureux pour le patient.

Par ailleurs, ces implants ont une tête relativement épaisse et étroite, destinée à venir se loger dans une cavité de forme complémentaire préalablement formée dans le trapèze. Cette coopération entre une tête épaisse et étroite et une cavité profonde et étroite donne de la stabilité à l'implant - c'est d'ailleurs le but recherché - car la tête est retenue dans la cavité, mais ne respecte pas la courbure anatomique de l'articulation trapézo-métacarpienne, qui est adaptée pour des mouvements relatifs amples entre le trapèze et l'extrémité proximale du métacarpe. A titre d'illustration, la figure 3 représente l'implant condylien de Swanson avec sa tige 5a insérée dans le métacarpe 2 du pouce et sa tête 5b logée dans une cavité profonde et étroite du trapèze 6. Le faible rayon de courbure de la partie de surface de la tête en contact avec le trapèze, rendu nécessaire par l'épaisseur et l'étroitesse de la tête, limite le débattement angulaire de l'implant, ce qui bride les mouvements du pouce.

### OBJECTIFS ET RESUME DE L'INVENTION

La présente invention vise notamment à fournir un implant trapézien ou trapézo-métacarpien qui soit au moins aussi stable que les implants évoqués ci-dessus et qui soit relativement facile à mettre en place.

A cette fin, il est prévu un implant trapézien ou trapézo-métacarpien comprenant une tige terminée par une tête, la tige et la tête étant destinées, respectivement, à être insérée dans le métacarpe du pouce et à être logée dans un espace obtenu notamment en retirant au moins une partie du trapèze, la surface de la tête étant constituée par une base reliée à la tige et destinée à reposer sur l'extrémité proximale du métacarpe, une partie de surface distale opposée à la base et servant de surface de contact avec l'os situé en regard de l'extrémité proximale du métacarpe, et une partie de raccord reliant la base et la partie de surface distale de la tête, **caractérisé en ce que** la partie de surface distale de la tête est inclinée par rapport à un axe longitudinal de la tige d'un angle prédéterminé, de façon que, lorsque la tige est montée droite dans le métacarpe, la partie de surface distale de la tête puisse être située en varus. Ledit os en regard de l'extrémité proximale du métacarpe est le scaphoïde dans le cas d'un implant trapézien, et la partie restante du trapèze dans le cas d'un implant trapézo-métacarpien.

Ainsi, selon l'invention, la stabilité de l'implant est donnée par le positionnement en varus de la partie de surface distale de la tête, comme dans l'article précité de Y. Allieu et al "L'implant trapézien de Swanson dans le traitement de l'arthrose péri-trapézienne". Toutefois, à la différence de cet article, ce n'est pas l'implant tout entier qui est positionné en varus, mais une partie seulement de l'implant, incluant la partie de surface distale de la tête.

L'inclinaison de la partie de surface distale de la tête par rapport à la tige permet d'insérer la tige de façon droite dans le métacarpe, c'est-à-dire suivant l'axe longitudinal de celui-ci. La tige peut donc être placée exclusivement dans la partie centrale molle de l'os, ce qui facilite le travail du chirurgien qui n'a pas à creuser dans la partie périphérique dure. Un tel positionnement de la tige évite en outre de fragiliser l'os dans une trop grande mesure.

Il n'est nullement besoin, selon l'invention, de ligaturer l'implant ou de le faire traverser par un tendon. D'autre part, contrairement aux implants trapézo-métacarpiens de la technique antérieure, la tête peut être large et sa partie de surface distale peut avoir un grand rayon de courbure correspondant sensiblement au rayon de courbure de l'articulation trapézo-métacarpienne. La tête peut également être plus plate. Le chirurgien n'a donc pas à faire une coupe trop importante sur l'extrémité proximale du métacarpe, ni à creuser profondément dans le trapèze, pour lequel le retrait d'une petite calotte de grand rayon de courbure peut suffire.

La stabilité de l'implant selon l'invention peut encore être augmentée en concevant ce dernier de telle sorte que la partie de surface distale de la tête soit non seulement inclinée mais également décalée par rapport à l'axe longitudinal de la tige du côté de l'implant où l'angle entre la partie de surface distale de la tête et l'axe longitudinal de la tige est le plus petit.

Au moins la partie de surface distale de la tête est conçue en pyrocarbone. Le pyrocarbone présente un très bon coefficient de frottement avec l'os, qui lui permet de glisser sans adhérence sur les os avec lesquels il est en contact et sans engendrer d'usure. A la différence du silicone, qui est trop mou, et du titane, qui est trop dur, le pyrocarbone présente un module d'élasticité, dit également module d'Young, voisin de celui de l'os. Les forces réciproques exercées sur l'implant et les os avoisinants se répartissent donc équitablement, réduisant ainsi le risque de douleur pour le patient.

### BREVE DESCRIPTION DES DESSINS

Dans les dessins annexés :
- les figures 1A, 1B et 1C, déjà commentées, représentent une partie d'une main dans laquelle est implanté un implant trapézien selon la technique antérieure, respectivement en position de repos, en position de pince avec implant subluxé et en position de pince avec implant luxé, cette partie de main étant vue du côté dorsal, avec le pouce et l'articulation trapézo-métacarpienne de profil et les autres doigts vus de dessus ;
- la figure 2, déjà commentée, représente, dans le même type de vue que celle des figures 1A, 1B et 1C, un implant trapézien selon la technique antérieure monté de façon oblique dans le métacarpe du pouce ;
- la figure 3, déjà commentée, représente, dans le même type de vue que celle des figures 1A, 1B et 1C, un implant trapézo-métacarpien selon la technique antérieure monté dans le métacarpe du pouce ;
- la figure 4A représente en vue de profil un implant trapézo-métacarpien selon un premier mode de réalisation de invention ;
- la figure 4B représente en vue de profil une variante de l'implant selon le premier mode de réalisation de l'invention ;
- la figure 5 montre, dans le même type de vue que celles des figures 1A, 1B et 1C, l'implant selon le premier mode de réalisation de l'invention mis en place dans la main d'un patient ;
- la figure 6 représente en vue de profil un implant trapézo-métacarpien selon un second mode de réalisation de l'invention ;
- la figure 7 représente en vue de profil un implant trapézien selon un troisième mode de réalisation de l'invention ; et
- la figure 8 représente en vue de profil un implant trapézien selon un quatrième mode de réalisation de l'invention.

### DESCRIPTION DETAILLEE DE PLUSIEURS MODES DE REALISATION DE L'INVENTION

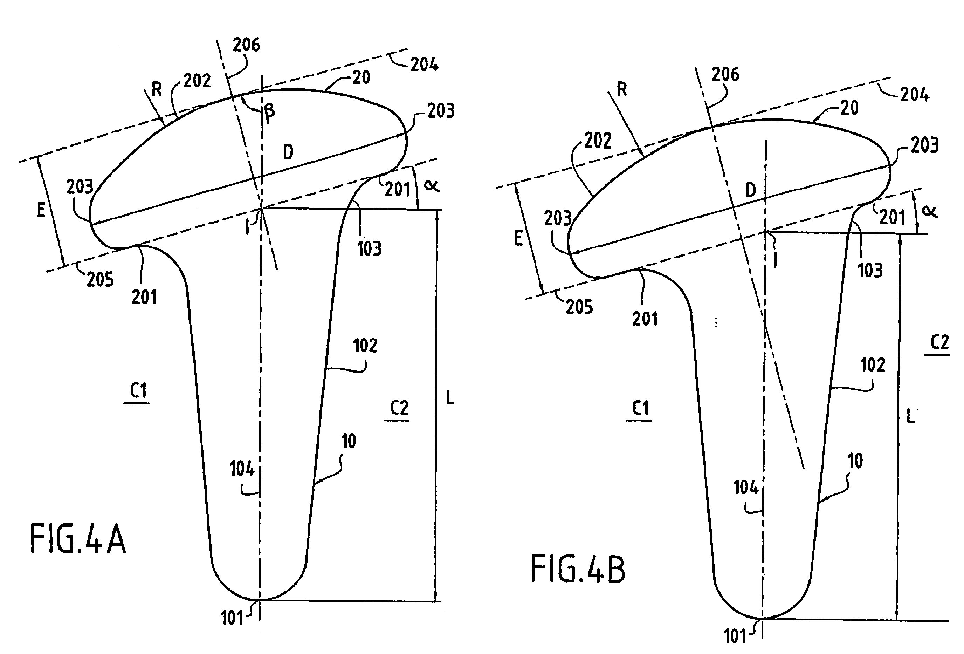

La figure 4A représente un implant selon un premier mode de réalisation de l'invention.

L'implant selon ce premier mode de réalisation est un implant trapézo-métacarpien, se présentant sous la forme générale d'un clou. Il comporte une tige 10 terminée, à une extrémité dite proximale, par une tête 20 et dont l'extrémité opposée, dite distale, est constituée par une pointe 101.

La tige 10 est destinée à être insérée dans le métacarpe du pouce, et la tête 20 à être logée dans un espace obtenu d'une part en pratiquant une coupe, ou résection, sur l'extrémité proximale du métacarpe et d'autre part en retirant une partie du trapèze en regard de la partie proximale du métacarpe.

La tête 20 a une forme aplatie. Sa section longitudinale est circulaire. Sa surface est constituée par une base annulaire et plane 201 reliée à l'extrémité proximale de la tige 10 et destinée à reposer sur la partie proximale du métacarpe, une partie de surface distale 202 opposée à la base 201 et servant de surface de contact et de frottement avec la partie restante du trapèze, et un bord latéral arrondi et annulaire 203 raccordant la partie de surface distale 202 à la base 201. Parmi les parties de surface 201, 202 et 203, seule la partie distale 202 est susceptible d'être en contact avec la partie restante du trapèze. La partie de surface distale 202 a une forme convexe, et consiste de préférence en une calotte sphérique.

La tige 10 comprend une partie tronconique 102 dont l'extrémité de plus petit diamètre est reliée à la pointe 101 et l'extrémité de plus grand diamètre est prolongée par une partie de raccord divergente 103 reliant la base 201 de la tête 20 à la partie tronconique 102 de la tige 10.

Conformément à l'invention, la partie de surface distale 202 de la tête 20 est inclinée par rapport à un axe longitudinal 104 de la tige 10 d'un angle prédéterminé α, c'est-à-dire que le plan tangent moyen 204 à la partie de surface distale 202 fait avec l'axe longitudinal 104 de la tige 10 un angle β=90°-α différent de 90°. Dans le mode de réalisation illustré à la figure 4A, la tête 20 dans sa totalité est inclinée par rapport à l'axe 104 de la tige 10. Ainsi, en particulier, la base 201 est inclinée par rapport à l'axe 104 dans le même sens que la partie de surface distale 202, et de l'angle α. L'angle d'inclinaison α est typiquement au moins égal à 10° et de préférence égal à 15°.

Le diamètre D de la tête 20 et le rayon de courbure R de la partie de surface distale 202 sont choisis suffisamment grands pour respecter le mieux possible la courbure anatomique de l'articulation trapézo-métacarpienne. Typiquement, l'implant selon l'invention est conçu en quatre tailles différentes. Dans chacune de ces tailles, le rapport entre le diamètre D de la tête, mesuré selon un axe longitudinal de la tête incliné par rapport à l'axe longitudinal 104 de la tige dans le même sens que la partie de surface distale 202 et d'un angle égal à l'angle α, et la longueur L de la tige, mesurée suivant l'axe longitudinal 104 de la tige entre l'extrémité distale 101 et le point d'intersection I entre le plan 205 défini par la base 201 et l'axe 104, est avantageusement au moins égal à 0,84. Le rapport entre le rayon de courbure R de la partie de surface distale 202 et la longueur L de la tige est avantageusement au moins égal à 0,6.

L'épaisseur E de la tête peut être relativement faible. Typiquement, le rapport entre l'épaisseur E de la tête 20, mesurée perpendiculairement à la base 201, et la longueur L de la tige est au plus égal à 0,42.

De préférence, la partie de surface distale 202 de la tête 20 est non seulement inclinée, mais également décalée par rapport à l'axe longitudinal 104 de la tige 10 du côté de l'implant où l'angle entre la partie de surface distale 202 et l'axe 104 est le plus petit, c'est-à-dire le côté désigné à la figure 4A par le repère C1 et correspondant à l'angle β=90°-α. Dans ce premier mode de réalisation, toute la tête 20 est décalée par rapport à l'axe 104 du côté C1. Ainsi, le centre géométrique de la tête 20, comme le centre de la partie de surface distale 202, est situé hors de l'axe 104, du côté C1.

Dans l'exemple illustré à la figure 4A, où la partie de surface distale 202 a une forme sphérique, l'axe de symétrie 206 de la partie de surface distale 202 coupe l'axe longitudinal 104 de la tige 10 au point I d'intersection entre le plan 205 défini par la base 201 de la tête 20 et l'axe 104. Toutefois, en variante, la partie de surface distale 202 peut être davantage décalée par rapport à la tige 10. La figure 4B illustre une telle configuration, dans laquelle l'axe de symétrie 206 de la partie de surface distale 202 coupe l'axe longitudinal 104 de la tige 10 en un point situé entre l'extrémité distale 101 de la tige 10 et le point I d'intersection entre le plan 205 défini par la base 201 et l'axe 104.

L'implant selon l'invention, constitué par la tige 10 et la tête 20, ou au moins la partie de surface distale 202 de la tête 20, est conçu en pyrocarbone. Selon une variante préférentielle, implant est formé d'un substrat en graphite revêtu d'une couche de pyrocarbone.

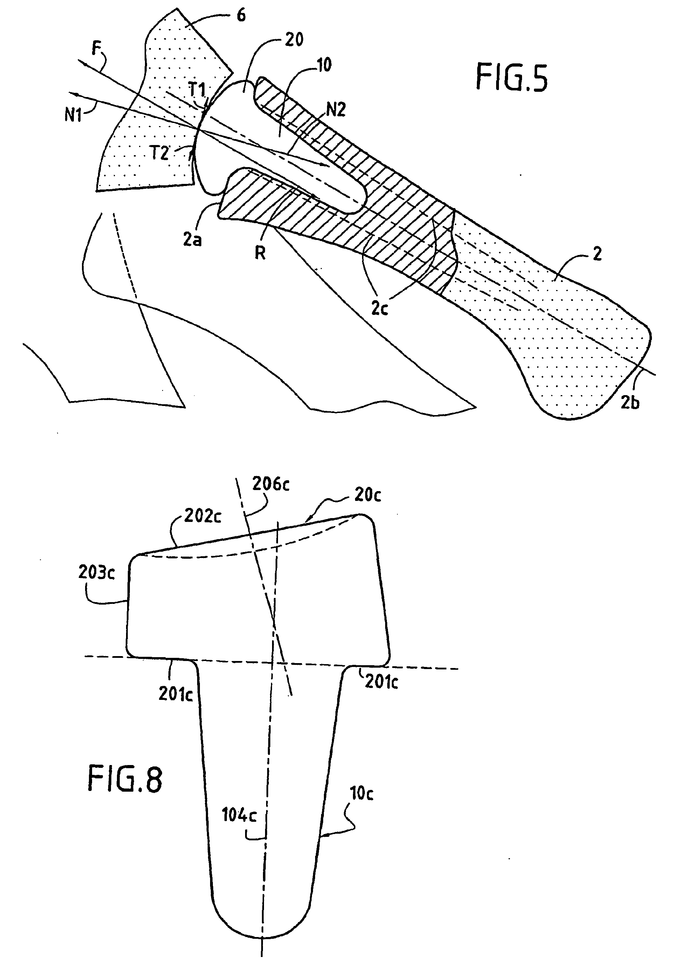

En référence à la figure 5, l'implant selon l'invention est mis en place de la façon suivante :
Une coupe oblique en varus est pratiquée dans l'extrémité proximale 2a du métacarpe 2 et une partie du trapèze 6 située en regard de cette extrémité proximale 2a est retirée, pour ménager un espace apte à accueillir la tête 20. Puis un trou droit est formé dans le fût du métacarpe, suivant l'axe longitudinal 2b de celui-ci. La tige 10 de l'implant est ensuite entrée à force dans le trou jusqu'à ce que la base inclinée 201 de la tête 20 vienne en appui sur la coupe inclinée 2a du métacarpe, de sorte que la tête 20 vienne se loger dans l'espace précité, en position de varus.
La tige 10 de l'implant est ainsi positionnée droite dans la partie centrale molle du métacarpe, délimitée à la figure 5 par les traits pointillés 2c, et est entourée par la partie périphérique dure de celui-ci. La mise en place de l'implant n'est donc pas plus compliquée que pour l'implant trapézien de Swanson illustré aux figures 1A, 1B et 1C. En outre, une fois mis en place comme décrit ci-dessus, l'implant est plus stable que l'implant trapézien de Swanson. Cette stabilité de l'implant selon l'invention est due principalement au fait que la surface de l'implant en contact avec le trapèze est augmentée par la position en varus de la partie de surface distale 202. La figure 5 illustre le bilan des forces exercées entre l'implant selon l'invention et le trapèze lorsque le patient effectue un effort de pince en tenant fermement un objet entre le pouce et l'index. Dans cette configuration, l'implant exerce sur le trapèze une force F ayant une composante T1 tangentielle à la surface de contact et une composante N1 normale à cette surface de contact. La grande surface de contact entre le trapèze et la tête de l'implant permet au trapèze d'exercer en réponse une force de réaction R ayant une composante tangentielle T2 suffisante pour contrebalancer la composante tangentielle T1 de la force F. Cet équilibre des forces maintient l'implant en contact avec le trapèze et réduit donc significativement le risque de luxation.

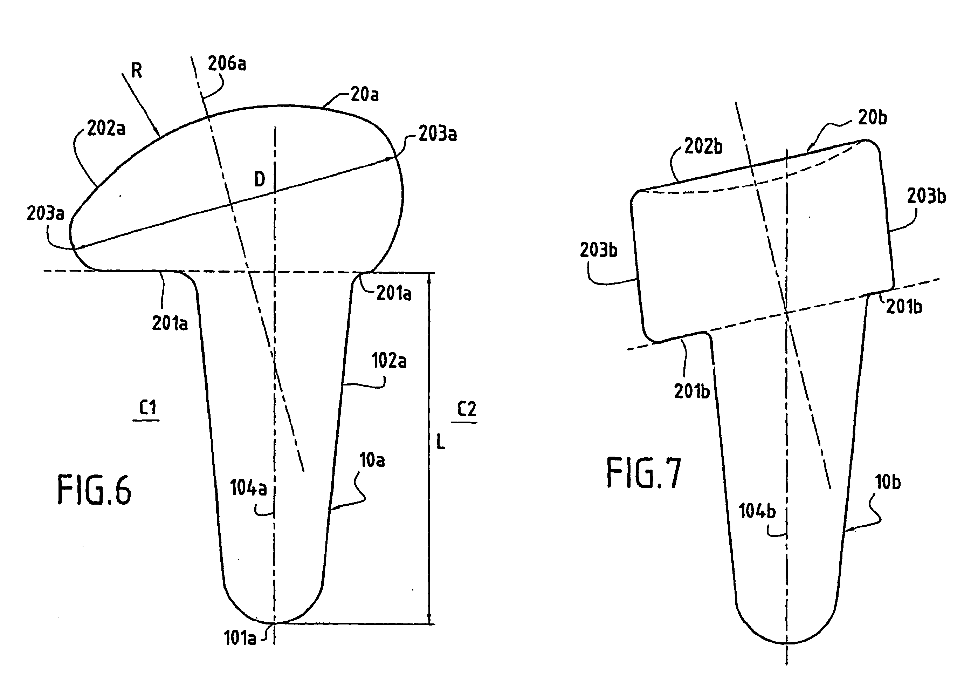

La figure 6 montre un second mode de réalisation de l'implant selon l'invention. L'implant selon ce second mode de réalisation est un implant trapézo-métacarpien qui diffère de l'implant selon le premier mode de réalisation essentiellement en ce que seule la partie de surface distale 202a est inclinée par rapport à la tige 10a, et non pas la tête 20a tout entière. Ainsi, la base 201 a de la tête 20a est perpendiculaire à l'axe longitudinal 104a de la tige 10a. La partie de raccord 203a de la tête 20a est donc plus haute du côté C2 de l'implant où l'angle entre la partie de surface distale 202a et l'axe 104a est le plus grand, que du côté opposé C1.

Comme dans le premier mode de réalisation, la partie de surface distale 202a est de préférence décalée par rapport à l'axe 104a de la tige du côté C1.

Les rapports indiqués plus haut entre le diamètre D de la tête et la longueur L de la tige et entre le rayon de courbure R de la partie de surface distale de la tête et la longueur L de la tige valent également pour ce second mode de réalisation. En revanche, l'épaisseur de la tête est nécessairement plus élevée.

Lorsqu'il est mis en place dans le patient, cet implant repose sur une coupe droite préalablement pratiquée dans l'extrémité proximale du métacarpe, et non pas sur une coupe oblique comme dans le premier mode de réalisation.

La figure 7 montre un troisième mode de réalisation de l'invention. L'implant selon ce troisième mode de réalisation est un implant trapézien, c'est-à-dire que sa tête 20b a une forme et une taille adaptées pour remplacer tout le trapèze. La partie de surface distale 202b de l'implant a une forme concave lui permettant de coopérer avec la partie distale du scaphoïde. La tête 20b, incluant la base 201 b, la partie de surface distale 202b et la partie de raccord 203b, est inclinée de façon comparable au premier mode de réalisation.

La figure 8 montre un quatrième mode de réalisation de l'invention. L'implant selon ce quatrième mode de réalisation est un implant trapézien dont la tête 20c comporte une partie de surface distale 202c inclinée par rapport à l'axe 104c de la tige 10c et une base 201 c perpendiculaire à l'axe 104c. Comme dans le second mode de réalisation, cet implant est destiné à reposer sur une coupe droite préalablement pratiquée dans l'extrémité proximale du métacarpe.

## Revendications

1. Implant trapézien ou trapézo-métacarpien comprenant une tige (10) terminée par une tête (20), la tige et la tête étant destinées, respectivement, à être insérée dans le métacarpe du pouce et à être logée dans un espace obtenu notamment en retirant au moins une partie du trapèze, la surface de la tête étant constituée par une base (201) reliée à la tige et destinée à reposer sur l'extrémité proximale du métacarpe, une partie de surface distale (202) opposée à la base et servant de surface de contact avec l'os situé en regard de l'extrémité proximale du métacarpe, et une partie de raccord (203) reliant la base et la partie de surface distale de la tête, **caractérisé en ce que** la partie de surface distale (202) de la tête est inclinée par rapport à un axe longitudinal (104) de la tige d'un angle prédéterminé (α), de façon que, lorsque la tige (10) est montée dans le métacarpe suivant l'axe longitudinal de celui-ci, la partie de surface distale (202) de la tête puisse être située en varus, et **en ce qu'**au moins la partie de surface distale (202) de la tête (20) est conçue en pyrocarbone.

2. Implant selon la revendication 1, **caractérisé en ce que** l'angle prédéterminé (α) est au moins égal à environ 10°.

3. Implant selon la revendication 1, **caractérisé en ce que** l'angle prédéterminé (α) est égal à environ 15°.

4. Implant selon l'une quelconque des revendications 1 à 3, **caractérisé en ce que** la partie de surface distale (202) de la tête est en outre décalée par rapport à l'axe longitudinal (104) de la tige du côté (C1) de l'implant où l'angle (β) entre la partie de surface distale (202) de la tête et l'axe longitudinal (104) de la tige est le plus petit.

5. Implant selon l'une quelconque des revendications 1 à 4, **caractérisé en ce que** la partie de surface distale (202) de la tête présente un axe de symétrie (206) faisant l'angle prédéterminé (α) avec l'axe longitudinal (104) de la tige.

6. Implant selon l'une quelconque des revendications 1 à 5, **caractérisé en ce que** la base (201) de la tête est inclinée par rapport à l'axe longitudinal (104) de la tige dans le même sens que la partie de surface distale (202), et d'un angle égal audit angle prédéterminé (α).

7. Implant selon les revendications 5 et 6, **caractérisé en ce que** l'axe de symétrie (206) de la partie de surface distale (202) de la tête coupe l'axe longitudinal (104) de la tige sensiblement au point d'intersection (I) entre un plan (205) défini par la base (201) de la tête et l'axe longitudinal (104) de la tige.

8. Implant selon les revendications 5 et 6, **caractérisé en ce que** l'axe de symétrie (206) de la partie de surface distale (202) de la tête coupe l'axe longitudinal (104) de la tige en un point situé entre une extrémité distale (101) de la tige et le point d'intersection (I) entre un plan (205) défini par la base (201) de la tête et l'axe longitudinal (104) de la tige.

9. Implant selon l'une quelconque des revendications 1 à 5, **caractérisé en ce que** la base (201a ; 201c) de la tête est sensiblement perpendiculaire à l'axe longitudinal (104a ; 104c) de la tige.

10. Implant selon l'une quelconque des revendications 1 à 9, **caractérisé en ce qu'**il consiste en un implant trapézo-métacarpien.

11. Implant selon la revendication 10, **caractérisé en ce que** la partie de surface distale (202) de la tête a une forme convexe.

12. Implant selon la revendication 10 ou 11, **caractérisé en ce que** le rapport entre le diamètre (D) de la tête, mesuré selon un axe longitudinal de la tête incliné par rapport à l'axe longitudinal (104) de la tige dans le même sens que la partie de surface distale (202) et d'un angle égal audit angle prédéterminé (α), et la longueur (L) de la tige, mesurée suivant l'axe longitudinal (104) de la tige entre une extrémité distale (101) de la tige et le point d'intersection (I) entre un plan (205) défini par la base (201) de la tête et l'axe longitudinal (104) de la tige, est au moins égal à 0,84.

13. Implant selon l'une quelconque des revendications 10 à 12, **caractérisé en ce que** le rapport entre un rayon de courbure (R) de la partie de surface distale (202) de la tête et la longueur (L) de la tige, mesurée suivant l'axe longitudinal (104) de la tige entre une extrémité distale (101) de la tige et le point d'intersection (I) entre un plan (205) défini par la base (201) de la tête et l'axe longitudinal (104) de la tige, est au moins égal à 0,6.

14. Implant selon l'une quelconque des revendications 10 à 13 lorsqu'elle dépend au moins de la revendication 6, **caractérisé en ce que** le rapport entre l'épaisseur (E) de la tête et la longueur (L) de la tige, mesurée suivant l'axe longitudinal (104) de la tige entre une extrémité distale (101) de la tige et le point d'intersection (I) entre un plan (205) défini par la base (201) de la tête et l'axe longitudinal (104) de la tige, est au plus égal à 0,42.

## Claims

1. Trapezal or trapezo-metacarpal implant comprising a stem (10) terminating in a head (20), the stem and the head being adapted respectively to be inserted in the metacarpus of the thumb and to be disposed in a space obtained particularly by removing at least a portion of the trapezium, the surface of the head being constituted by a base (201) connected to the stem and adapted to rest on the proximal end of the metacarpus, a distal surface portion (202) opposite the base and serving as a contact surface with the bone located facing the proximal end of the metacarpus, and a connecting portion (203) connecting the base and the distal surface portion of the head, **characterised in that** the distal surface portion (202) of the head is inclined relative to a longitudinal axis (104) of the stem by a predetermined angle (α), such that, when the stem (10) is mounted in the metacarpus along the longitudinal axis thereof, the distal surface portion (202) of the head can be located in varus, and **in that** at least the distal surface portion (202) of the head (20) is of pyrocarbon.

2. Implant according to claim 1, **characterised in that** the predetermined angle (α) is at least equal to about 10°.

3. Implant according to claim 1, **characterised in that** the predetermined angle (α) is equal to about 15°.

4. Implant according to any one of claims 1 to 3, **characterised in that** the distal surface portion (202) of the head is moreover offset relative to the longitudinal axis (104) of the stem to the side (C1) of the implant where the angle (β) between the distal surface portion (202) of the head and the longitudinal axis (104) of the stem is smaller.

5. Implant according to any one of claims 1 to 4, **characterised in that** the distal surface portion (202) of the head has an axis of symmetry (206) forming the predetermined angle (α) with the longitudinal axis (104) of the stem.

6. Implant according to any one of claims 1 to 5, **characterised in that** the base (201) of the head is inclined relative to the longitudinal axis (104) of the stem in the same direction as the distal surface portion (202), and by an angle equal to said predetermined angle (α).

7. Implant according to claims 5 and 6, **characterised in that** the axis of symmetry (206) of the distal surface portion (202) of the head intersects the longitudinal axis (104) of the stem substantially at the point of intersection (I) between a plane (205) defined by the base (201) of the head and the longitudinal axis (104) of the stem.

8. Implant according to claims 5 and 6, **characterised in that** the axis of symmetry (206) of the distal surface portion (202) of the head intersects the longitudinal axis (104) of the stem at a point located between a distal end (101) of the stem and the point of intersection (I) between a plane (205) defined by the base (201) of the head and the longitudinal axis (104) of the stem.

9. Implant according to any one of claims 1 to 5, **characterised in that** the base (201 a; 201 c) of the head is substantially perpendicular to the longitudinal axis (104a; 104c) of the stem.

10. Implant according to any one of claims 1 to 9, **characterised in that** it consists of a trapezo-metacarpal implant.

11. Implant according to claim 10, **characterised in that** the distal surface portion (202) of the head has a convex shape.

12. Implant according to claim 10 or 11, **characterised in that** the ratio between the diameter (D) of the head, measured along a longitudinal axis of the head inclined relative to the longitudinal axis (104) of the stem in the same direction as the distal surface portion (202) and by an angle equal to said predetermined angle (α), and the length (L) of the stem, measured along the longitudinal axis (104) of the stem between a distal end (101) of the stem and the point of intersection (I) between a plane (205) defined by the base (201) of the head and the longitudinal axis (104) of the stem, is at least equal to 0.84.

13. Implant according to any one of claims 10 to 12, **characterised in that** the ratio between a radius of curvature (R) of the distal surface portion (202) of the head and the length (L) of the stem, measured along the longitudinal axis (104) of the stem between a distal end (101) of the stem and the point of intersection (I) between a plane (205) defined by the base (201) of the head and the longitudinal axis (104) of the stem, is at least equal to 0.6.

14. Implant according to any one of claims 10 to 13 when it depends at least from claim 6, **characterised in that** the ratio between the thickness (E) of the head and the length (L) of the stem, measured along the longitudinal axis (104) of the stem between a distal end (101) of the stem and the point of intersection (I) between a plane (205) defined by the base (201) of the head and the longitudinal axis (104) of the stem, is at most equal to 0.42.

## Patentansprüche

1. Trapezium- oder trapezio-metakarpales Implantat, welches einen in einen Kopf (20) endenden Schaft (10) umfasst, wobei der Schaft und der Kopf dazu bestimmt sind, in den Mittelhandknochen des Daumens eingesetzt zu werden bzw. in einem Zwischenraum aufgenommen zu werden, welcher insbesondere durch Entfernen mindestens eines Teils des Trapeziums erhalten wird, wobei die Oberfläche des Kopfes aus einer Basis (201) besteht, die mit dem Schaft verbunden ist und dazu bestimmt ist, auf dem proximalen Ende des Mittelhandknochens aufzuliegen, sowie aus einem der Basis gegenüberliegenden distalen Oberflächenabschnitt (202), der als Kontaktfläche für den dem proximalen Ende des Mittelhandknochens zugewandten Knochen dient, und aus einem Verbindungsteil (203), welcher die Basis und den distalen Oberflächenabschnitt des Kopfes verbindet, **dadurch gekennzeichnet, dass** der distale Oberflächenabschnitt (202) des Kopfes in Bezug auf eine Längsachse (104) des Schaftes um einen vorbestimmten Winkel (α) geneigt ist, so dass bei in dem Mittelhandknochen dessen Längsachse folgend angebrachtem Schaft (10) sich der distale Oberflächenabschnitt (202) des Kopfes in einer Varusstellung befinden kann, und **dadurch**, dass zumindest der distale Oberflächenabschnitt (202) des Kopfes (20) aus Pyrocarbon ausgebildet ist.

2. Implantat nach Anspruch 1, **dadurch gekennzeichnet, dass** der vorbestimmte Winkel (α) mindestens gleich etwa 10° ist.

3. Implantat nach Anspruch 1, **dadurch gekennzeichnet, dass** der vorbestimmte Winkel (α) gleich etwa 15° ist.

4. Implantat nach einem beliebigen der Ansprüche 1 bis 3, **dadurch gekennzeichnet, dass** der distale Oberflächenabschnitt (202) des Kopfes zudem auf der Seite (C1) des Implantats, wo der Winkel (β) zwischen dem distalen Oberflächenabschnitt (202) des Kopfes und der Längsachse (104) des Schaftes am kleinsten ist, in Bezug auf die Längsachse (104) des Schaftes versetzt ist.

5. Implantat nach einem beliebigen der Ansprüche 1 bis 4, **dadurch gekennzeichnet, dass** der distale Oberflächenabschnitt (202) des Kopfes eine Symmetrieachse (206) aufweist, welche den vorbestimmten Winkel (α) mit der Längsachse (104) des Schaftes bildet.

6. Implantat nach einem beliebigen der Ansprüche 1 bis 5, **dadurch gekennzeichnet, dass** die Basis (201) des Kopfes in Bezug auf die Längsachse (104) des Schaftes in der gleichen Richtung wie der distale Oberflächenabschnitt (202) und um einen dem vorbestimmten Winkel (α) gleichen Winkel geneigt ist.

7. Implantat nach den Ansprüchen 5 und 6, **dadurch gekennzeichnet, dass** die Symmetrieachse (206) des distalen Oberflächenabschnitts (202) des Kopfes die Längsachse (104) des Schaftes im Wesentlichen in dem Schnittpunkt (I) zwischen einer durch die Basis (201) des Kopfes definierten Ebene (205) und der Längsachse (104) des Schaftes schneidet.

8. Implantat nach den Ansprüchen 5 und 6, **dadurch gekennzeichnet, dass** die Symmetrieachse (206) des distalen Oberflächenabschnitts (202) des Kopfes die Längsachse (104) des Schaftes in einem Punkt schneidet, welcher sich zwischen einem distalen Ende (101) des Schaftes und dem Schnittpunkt (I) zwischen einer durch die Basis (201) des Kopfes definierten Ebene (205) und der Längsachse (104) des Schaftes befindet.

9. Implantat nach einem beliebigen der Ansprüche 1 bis 5, **dadurch gekennzeichnet, dass** die Basis (201 a; 201 c) des Kopfes im Wesentlichen senkrecht zu der Längsachse (104a; 104c) des Schaftes ist.

10. Implantat nach einem beliebigen der Ansprüche 1 bis 9, **dadurch gekennzeichnet, dass** es aus einem trapezio-metakarpalen Implantat besteht.

11. Implantat nach Anspruch 10, **dadurch gekennzeichnet, dass** der distale Oberflächenabschnitt (202) des Kopfes eine konvexe Form aufweist.

12. Implantat nach Anspruch 10 oder 11, **dadurch gekennzeichnet, dass** das Verhältnis zwischen dem Durchmesser (D) des Kopfes, gemessen entlang einer Längsachse des Kopfes, welche in Bezug auf die Längsachse (104) des Schaftes in der gleichen Richtung wie der distale Oberflächenabschnitt (202) und um einen dem vorbestimmten Winkel (α) gleichen Winkel geneigt ist, und der Länge (L) des Schaftes, gemessen entlang der Längsachse (104) des Schaftes zwischen einem distalen Ende (101) des Schaftes und dem Schnittpunkt (I) zwischen einer durch die Basis (201) des Kopfes definierten Ebene (205) und der Längsachse (104) des Schaftes, mindestens gleich 0,84 ist.

13. Implantat nach einem beliebigen der Ansprüche 10 bis 12, **dadurch gekennzeichnet, dass** das Verhältnis zwischen einem Krümmungsradius (R) des distalen Oberflächenabschnitts (202) des Kopfes und der Länge (L) des Schaftes, gemessen entlang der Längsachse (104) des Schaftes zwischen einem distalen Ende (101) des Schaftes und dem Schnittpunkt (I) zwischen einer durch die Basis (201) des Kopfes definierten Ebene (205) und der Längsachse (104) des Schaftes, mindestens gleich 0,6 ist.

14. Implantat nach einem beliebigen der Ansprüche 10 bis 13, wenn dieser mindestens von Anspruch 6 abhängt, **dadurch gekennzeichnet, dass** das Verhältnis zwischen der Dicke (E) des Kopfes und der Länge (L) des Schaftes, gemessen entlang der Längsachse (104) des Schaftes zwischen einem distalen Ende (101) des Schaftes und dem Schnittpunkt (I) zwischen einer durch die Basis (201) des Kopfes definierten Ebene (205) und der Längsachse (104) des Schaftes, höchstens gleich 0,42 ist.
